# EUROPEAN PATENT APPLICATION

(11) **EP 4 129 218 A1**
(43) Date of publication of application: **08.02.2023**
(21) Application number: 21781701.4
(22) Date of filing: 18.02.2021
(51) Int. Cl.: A61B 17/34

(54) **DILATOR**

(30) Priority: 03.04.2020 JP 2020067688
(71) Applicant: Asahi Intecc Co., Ltd., Seto-shi, Aichi 489-0071 (JP)
(72) Inventor: TSUZUKU, Marina, Seto-shi, Aichi 489-0071 (JP); KOSUGI, Tomoki, Seto-shi, Aichi 489-0071 (JP); FUSEYA, Yukihiro, Seto-shi, Aichi 489-0071 (JP); KURITA, Daisuke, Seto-shi, Aichi 489-0071 (JP)
(74) Representative: Prinz & Partner mbB
(86) International application number: PCT/JP2021/006106
(87) International publication number: WO 2021/199763

(57) **Abstract**

An object is to provide a dilator capable of preventing a spirally-arranged protruding portion from being detached from a hollow shaft while suppressing a decrease in the flexibility of a tapered portion.

Dilator 1 includes a hollow shaft 11 having a tapered portion 111, in which the outer diameter of the distal end is smaller than the outer diameter of the proximal end, and a main body portion 112 which has a distal end located at the proximal end of the tapered portion 111, extends toward the proximal end side, and has a constant outer diameter, wherein: a spirally-arranged protruding portion 21 which is configured of a coil body 21C formed by winding a wire is provided on the outer peripheral surface 11s of the tapered portion 111 and the main body portion 112; the spirally-arranged protruding portion 21 has gaps 21g between portions adjacent to each other along the longitudinal direction of the hollow shaft 11; and the spirally-arranged protruding portion 21 is not joined to the tapered portion 111, but is joined to the main body portion 112 at a joint part B.

## Description

### TECHNICAL FIELD

The disclosed embodiments relate to a dilator.

### BACKGROUND ART

A dilator is known as, for example, an instrument for expanding a hole made in a body surface or an organ etc., or expanding a constricted part formed in a bile duct or a pancreatic duct, etc.

Such a dilator requires a strong propulsive force for expansion, and thus an example thereof disclosed (see, for example, Patent Literature 1) is a dilator that is provided with a coil body on the outer peripheral surface of the tapered portion of a shaft for expansion, so as to supplement the propulsive force by the use of the screwing action thereof.

The coil body as described above is configured by, for example, winding a wire around the outer peripheral surface of the above tapered portion, whereby the coil body can be easily formed.

### CITATION LIST

### Patent Literature

Patent Literature 1: WO 2013/038720

### SUMMARY OF INVENTION

### TECHNICAL PROBLEM

However, a conventional dilator as described above is configured such that a wire forming a coil body is simply wound around the outer peripheral surface of a shaft, and thus may cause a risk that the wire is displaced due to a drag or the like when expanding a hole, or the wire is detached from the shaft.

Meanwhile, a configuration in which a wire is joined to a shaft is also conceivable. However, since a dilator is also required to have flexibility for smooth advancement in a curved body cavity, easy joining causes a decrease in the flexibility of the shaft.

The disclosed embodiments have been made in view of the above circumstances, and an object of the present invention is to provide a dilator capable of preventing a spirally-arranged protruding portion from being detached from the hollow shaft while suppressing a decrease in the flexibility of a tapered portion.

### SOLUTION TO PROBLEM

Some aspects of this disclosure are as follows.
(1) A dilator including a hollow shaft having: a tapered portion in which the outer diameter of the distal end is smaller than the outer diameter of the proximal end and a main body portion which has a distal end located at the proximal end of the tapered portion, extends toward the proximal end side, and has a constant outer diameter, wherein
   a spirally-arranged protruding portion which is configured of a coil body formed by winding a wire is provided on the outer peripheral surface of the tapered portion and the main body portion,
   the spirally-arranged protruding portion has gaps between portions adjacent to each other along the longitudinal direction of the hollow shaft, and
   the spirally-arranged protruding portion is not joined to the tapered portion, but is joined to the main body portion at the joint part.
(2) The dilator according to (1) above, wherein the joint part is located at a position other than positions in the distal end portion of the main body portion.
(3) The dilator according to (1) or (2) above, wherein the joint part is located at only one position.
(4) The dilator according to (3) above, wherein the joint part is located at the proximal end portion of the spirally-arranged protruding portion.
(5) The dilator according to (1) or (2) above, wherein the joint part is located at two or more positions, and all of the joint parts are located only within the range of one round of the spirally-arranged protruding portion.
(6) The dilator according to any one of (1) to (5) above, wherein the hollow shaft is configured of a coil body formed by winding a wire, and
(7) The dilator according to (6) above, wherein the wire of the coil body in the hollow shaft is configured of multiple solid wires,
   a midway portion and/or the proximal end portion of the hollow shaft in the longitudinal direction has a binding part in which the plurality of solid wires are integrally bound, and
   the joint part is located at a position other than the positions in the binding part.

Note that in the specification, the "distal end side" refers to a direction along the longitudinal direction of a dilator and refers to a direction along which the dilator is advanced toward a portion set to be expanded in diameter. The "proximal end side" is a direction along the longitudinal direction of the dilator, and refers to a direction opposite to the distal end side. Moreover, the "distal end" refers to an end on the distal end side of an arbitrary member or portion, and the "proximal end" refers to an end on the proximal end side of an arbitrary member or portion. Further, the "distal end portion" refers to a portion of an arbitrary member or portion, which includes its distal end and extends halfway from the distal end to the proximal end side. The "proximal end portion" refers to a portion of an arbitrary member or portion, which includes its proximal end and extends halfway from the proximal end to the distal end side.

### ADVANTAGEOUS EFFECTS OF INVENTION

The disclosed embodiments can provide a dilator capable of preventing a spirally-arranged protruding portion from being detached from a hollow shaft while suppressing a decrease in the flexibility of a tapered portion.

### BRIEF DESCRIPTION OF DRAWINGS

FIG. 1 is a schematic side view illustrating a first embodiment.
FIG. 2A is a schematic side view illustrating a modified example of the first embodiment.
FIG. 2B is a schematic side view illustrating a modified example of the first embodiment.
FIG. 2C is a schematic side view illustrating a modified example of the first embodiment.
FIG. 2D is a schematic side view illustrating a modified example of the first embodiment.
FIG. 3 is a schematic side view illustrating a second embodiment.
FIG. 4 is a schematic side view illustrating a third embodiment.
FIG. 5A is a schematic side view illustrating a modified example of the third embodiment.
FIG. 5B is a schematic side view illustrating a modified example of the third embodiment.
FIG. 6 is a schematic side view illustrating a fourth embodiment.
FIG. 7 is a schematic side view illustrating a modified example of the first embodiment.
FIG. 8 is a schematic side view illustrating a modified example of the first embodiment.

### DESCRIPTION OF EMBODIMENTS

The dilator of the disclosure includes a hollow shaft having a tapered portion in which the outer diameter of the distal end is smaller than the outer diameter of the proximal end and a main body portion which has a distal end located at the proximal end of the tapered portion, extends toward the proximal end side, and has a constant outer diameter. A spirally-arranged protruding portion which is configured of a coil body formed by winding a wire is provided on the outer peripheral surface of the tapered portion and the main body portion. The spirally-arranged protruding portion has gaps between portions adjacent to each other along the longitudinal direction of the hollow shaft. The spirally-arranged protruding portion is not joined to the tapered portion, but is joined to the main body portion at the joint part.

Hereinafter, the first to fourth embodiments of the disclosed embodiments are described with reference to the enclosed drawings. However, the disclosed embodiments are not limited to the embodiments illustrated in the drawings. Moreover, the size of the dilator in each drawing is a size illustrated to facilitate understanding of the embodiments, and does not correspond to the actual size.

### [First Embodiment]

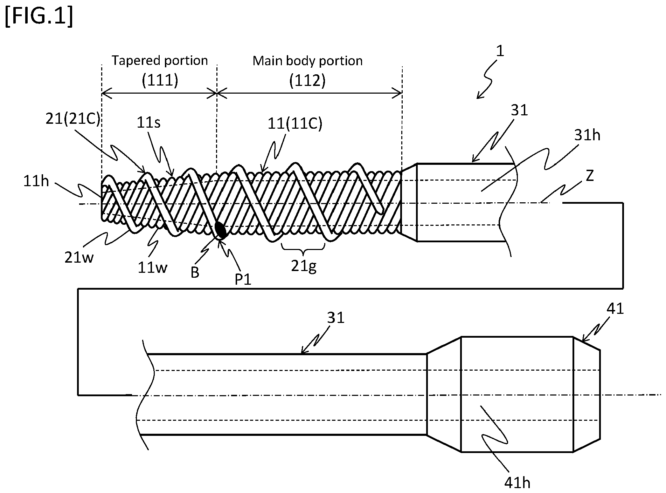

FIG. 1 is a schematic side view illustrating the first embodiment. A dilator 1 is, as shown in FIG. 1, roughly configured of a hollow shaft 11, a spirally-arranged protruding portion 21, a proximal end side shaft 31, and a base portion 41.

The hollow shaft 11 is a hollow-shaped shaft having a through-hole 11h (a portion inside the area flanked by common inscribed lines illustrated with the broken lines in FIG. 1). The through-hole 11h is, for example, a through hole through which a guide wire (not shown) or the like is inserted, and is configured of a continuous space connecting the distal end and the proximal end of the hollow shaft 11 so that the guide wire or the like can be freely inserted. The hollow shaft 11 has a tapered portion 111 and a main body portion 112.

The tapered portion 111 is a portion in which the outer diameter of the distal end is smaller than the outer diameter of the proximal end. Specifically, for example, the tapered portion 111 is connected to the distal end of the main body portion 112 described later, extends from the distal end of the main body portion 112 toward the distal end side, and has a shape that tapers toward the distal end side.

The main body portion 112 is a portion, which has a distal end located at the proximal end of the tapered portion 111, extends toward the proximal end side, and has a constant outer diameter. Specifically, for example, the main body portion 112 can be configured such that the distal end continues to the proximal end of the tapered portion 111 and the proximal end continues to the proximal end side shaft 31 described later.

The tapered portion 111 and the main body portion 112 described above may be integrally formed or may be formed as separate bodies. The hollow shaft 11 of the embodiment is configured of a coil body 11C, wherein the tapered portion 111 and the main body portion 112 are integrally formed and a wire 11w made of one solid wire is continuously and spirally wound around the long axis Z of the dilator 1 to form the coil body 11C. Since the hollow shaft 11 is configured of the coil body 11C formed by winding the wire 11w in this way, torquability (the certainty of transmitting the rotational force applied to the proximal end portion of the dilator to the distal end portion) can be increased.

Materials forming the hollow shaft 11 is preferably antithrombotic, flexible, and biocompatible because the dilator 1 is to be inserted into a body cavity. Examples of the above materials that can be employed herein include resin materials such as a polyamide resin, a polyolefin resin, a polyester resin, a polyurethane resin, a silicone resin, and a fluororesin, and metal materials such as stainless steel and superelastic alloys (a nickel-titanium alloy). The materials forming the tapered portion and the main body portion may be the same materials or different materials.

The coil body 11C of the tapered portion 111 and the main body portion 112 may be formed integrally or separately. Note that the coil body 11C of the tapered portion 111 and the main body portion 112 may be formed of the same or different materials. Further, the wire diameters of the coil body 11C of the tapered portion 111 and the main body portion 112 may be the same or different. The coil body 11C of the tapered portion 111 and the main body portion 112 in the embodiment is formed by spirally winding a wire 11w that is a solid wire made of the same material and having the same wire diameter.

Note that the hollow shaft 11 may have various coatings (not shown) in a side portion of the outer peripheral surface 11s thereof. Examples of the coatings include a protective film (plating film and the like) for protecting the surface of the hollow shaft 11, a base film for improving the adhesion between the hollow shaft 11 and the spirally-arranged protruding portion 21, and the like.

The spirally-arranged protruding portion 21 is a member configured of a coil body 21C which is provided on the outer peripheral surface 11s of the tapered portion 111 and the main body portion 112 and around which a wire 21w is wound. The spirally-arranged protruding portion 21 has gaps 21g between portions adjacent to each other along the longitudinal direction of the hollow shaft 11. Specifically, the spirally-arranged protruding portion 21 can be formed, for example, by spirally winding a continuous or intermittent single-thread or multi-thread wires 21w along the longitudinal direction of the hollow shaft 11 in such a manner that the wire(s) 21w is in contact with the outer peripheral surface 11s.

A wire(s) forming the spirally-arranged protruding portion 21 may be a solid wire or twisted wires, or a combination of a solid wire and twisted wires. Note that the "solid wire" refers to a single wire and the "twisted wire" refers to a bundle (group) of wires formed by twisting a plurality of single wires with each other in advance.

As materials forming the spirally-arranged protruding portion 21, for example, the same materials or the like as those exemplified as materials forming the hollow shaft 11 can be employed.

Here, the spirally-arranged protruding portion 21 is not joined to the tapered portion 111, but is joined to the main body portion 112 at the joint part B.

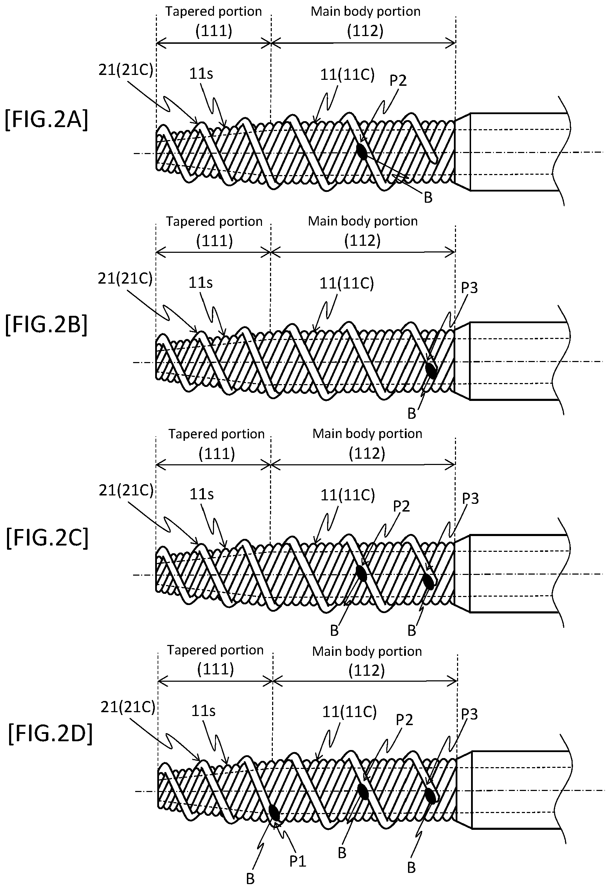

Examples of the position where the joint part B is provided include a position where the distal end portion of the main body portion 112 and the spirally-arranged protruding portion 21 come into contact (position P1, see FIG. 1), a position where a midway portion of the main body portion 112 and the spirally-arranged protruding portion 21 come into contact (position P2, see FIG. 2A), a position where the main body portion 112 and the proximal end portion of the spirally-arranged protruding portion 21 come into contact (position P3, see FIG. 2B), a combined position (for example, see FIG. 2C and FIG. 2D) of at least two positions from among position P1, position P2, and position P3 above, and the like. The embodiment illustrates a joint part B provided at the position P1 where the distal end portion of the main body portion 112 and the spirally-arranged protruding portion 21 come into contact with each other.

Examples of a method for joining the spirally-arranged protruding portion 21 and the main body portion 112, which can be employed herein, include a method for brazing the two via a brazing material, a method for directly welding the two, a method for adhering the two via an adhesive, and a method for fixing the spirally-arranged protruding portion 21 to a coating such as a base film. In the case of brazing, examples of the brazing material include silver tin wax and gold tin wax.

The joint part B is preferably located at a position other than positions in the distal end portion of the main body portion 112. In such a case, the joint part B can be provided, for example, at the position P2 and/or the position P3 described above (see, for example, FIGS. 2A, 2B, and 2C).

In this manner, when the joint part B is located at a position other than positions in the distal end portion of the main body portion 112, the flexibility of the distal end portion can be increased because the joint part B is not located at the distal end portion of the main body portion 112, and the flexibility in the distal end side of the hollow shaft 11 including the tapered portion 111 can be further increased.

It is also preferable that the joint part B is located at only one position. In such a case, the joint part B can be provided, for example, at any one position of the above-mentioned positions P1, P2, and P3 (see, for example, FIGS. 1, 2A, and 2B).

As described above, the joint part B is located at only one position, so as to prevent the spirally-arranged protruding portion 21 from being detached from the hollow shaft 11. Such a low number of the joint part B for joining the hollow shaft 11 and the spirally-arranged protruding portion 21 enables to suppress an increase in the rigidity of the hollow shaft 11 due to joining, and leads to reliable suppression of a decrease in the flexibility of the hollow shaft 11.

Further, if the joint part B is located at only one position, the joint part B is preferably located at only the proximal end portion of a spirally-arranged protruding portion. In such a case, the joint part B can be provided, for example, only at the position P3 described above (see, for example, FIG. 2B).

As described above, the joint part B is located only at the proximal end portion of the spirally-arranged protruding portion, so that an increase in the rigidity on the distal end side of the hollow shaft 11 can be suppressed as far as possible, while joining the hollow shaft 11 and the spirally arranged protruding portion 21.

The proximal end side shaft 31 is a shaft having a through-hole 31h that communicates with a through-hole 11h of the hollow shaft 11 and penetrates from the distal end to the proximal end. The distal end of the proximal end side shaft 31 is connected to, for example, the proximal end of the hollow shaft 11 and the proximal end of the proximal end side shaft 31 is connected to a base portion 41 described later.
The proximal end side shaft 31 may be a coil body formed of a wire that is continuously and spirally wound around the long axis Z of the dilator 1 (a state in which adjacent wire portions are in contact with each other along the longitudinal direction).

Examples of the material forming the proximal end side shaft 31 include stainless steel such as SUS304, a superelastic alloy such as a Ni-Ti alloy, and a cobalt chrome alloy. This can improve pushability of the hollow shaft 11 as a result of the operation of the base portion 41.

The base portion 41 is a portion where a technician operates the dilator 1. The base portion 41 has, for example, a through-hole 41h that communicates with the through-hole 31h of the proximal end side shaft 31 and penetrates from the distal end to the proximal end. The base portion 41 can be configured such that the distal end is connected to the proximal end of the proximal end side shaft 31. During the procedure, a guide wire or the like is inserted into the through-hole 41h, and a technician operates the base portion 41 to, for example, advance and retract the hollow shaft 11 or rotate the same, thereby expanding the hole.

Next, an example of the use mode of the dilator 1 is described as follows. Here, a procedure for expanding a portion to be expanded such as a chronic total occlusion (CTO) formed in a blood vessel using the dilator 1 is described.

First, use an introducer needle (not shown) to make a hole while puncturing a portion to be expanded. Next, after insertion of a guide wire (not shown) into the through-hole of the introducer needle, pull out the introducer needle. Next, insert the proximal end of the guide wire into the through-hole 11h from the distal end side of the hollow shaft 11, and push the hollow shaft 11 to the portion to be expanded. At this time, the dilator 1 can be smoothly advanced and retracted with the flexible tapered portion 111 while following the shape of the curved portion to be inserted (for example, blood vessel, esophagus, stomach, bile duct, etc.).

Next, after insertion of the tapered portion 111 into the hole made in the portion to be expanded, operate the base portion 41 to expand the hole by the tapered portion 111 while rotating and advancing the hollow shaft 11. At this time, a propulsive force is applied to the hollow shaft 11 by the screwing action of the spirally-arranged protruding portion 21 accompanied by the rotation of the hollow shaft 11, and the hole is gradually expanded by the outer peripheral surface 11s as the tapered portion 111 is advanced. Further, since a coil body 21C is joined to the main body portion 112 at the joint part B, the movement of the coil body 21C with respect to the hollow shaft 11 is restricted, thereby, for example, preventing the coil body 21C from being detached from the hollow shaft 11 due to a reaction applied to the coil body 21C when the portion to be expanded is expanded.

The dilator 1 is configured as described above, so as to be able to prevent the spirally-arranged protruding portion 21 from being detached from the hollow shaft 11 while suppressing a decrease in the flexibility of the tapered portion 111.

Here, although the hollow shaft 11 and the spirally-arranged protruding portion 21 are joined, a decrease in the flexibility of the tapered portion 111 can be suppressed. It is inferred that this is because the spirally-arranged protruding portion 21 is not joined to the tapered portion 111. Specifically, compared to a case where a joint part is provided on the outer peripheral surface of a tapered portion, in a case where no joint part is provided on a tapered portion, the movement of itself (tapered portion 111) is not restricted (its own degree of freedom is not lowered) by another member (spirally-arranged protruding portion 21), and thus it seems that a decrease in flexibility (an increase in rigidity) can be suppressed.

### [Second Embodiment]

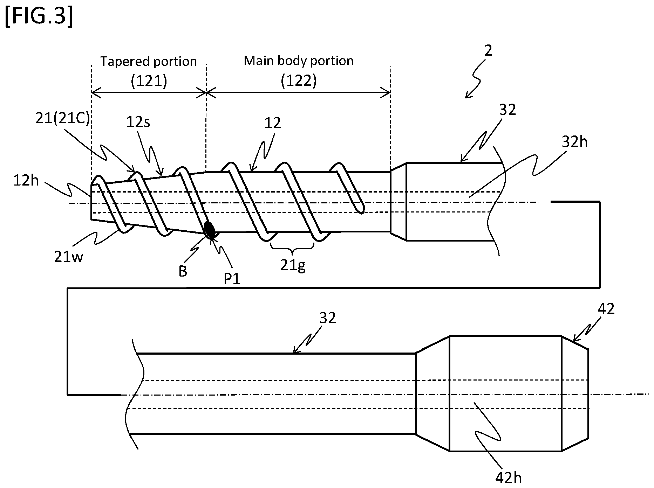

FIG. 3 is a schematic side view illustrating the second embodiment of the invention. A dilator 2 is roughly configured of a hollow shaft 12, a spirally-arranged protruding portion 21, a proximal end side shaft 32, and a base portion 42, as shown in FIG. 3. The dilator 2 differs from that of the first embodiment in that it includes the hollow shaft 12, the proximal end side shaft 32 and the base portion 42. Since the configuration of the spirally-arranged protruding portion 21 is the same as that of the first embodiment, the same portions/positions are represented by the same reference signs, and detailed descriptions thereof are omitted. Further, since the configurations other than the configurations of the hollow shaft 12, the proximal end side shaft 32, and the base portion 42 and the use mode of the dilator 2 shown below are the same as those of the first embodiment, detailed descriptions thereof are omitted.

The hollow shaft 12 is a hollow-shaped shaft having a through-hole 12h. The hollow shaft 12 has a tapered portion 121 and a main body portion 122. The tapered portion 121 is a portion in which the outer diameter of the distal end is smaller than the outer diameter of the proximal end. The main body portion 122 is a portion which has a distal end located at the proximal end of the tapered portion 121, extends toward the proximal end side, and has a constant outer diameter.

The tapered portion 121 and the main body portion 122 may be formed integrally or separately. Note that the tapered portion 121 and the main body portion 122 may be formed of the same or different materials. Further, the tapered portion 121 and the main body portion 122 may have the same or different wall thicknesses. The tapered portion 121 and the main body portion 122 of this embodiment are formed using the same materials integrally by casting or the like, and have different wall thicknesses.

The spirally-arranged protruding portion 21 is not joined to the tapered portion 121, but is joined to the main body portion 122 at the joint part B. The embodiment illustrates the dilator 2, wherein the joint part B is formed at a part (position P1) where the spirally-arranged protruding portion 21 and the distal end portion of the main body portion 122 contact to each other.

The proximal end side shaft 32 is a shaft having a through-hole 32h that communicates with a through-hole 12h of a hollow shaft 12 and penetrates from the distal end to the proximal end. The distal end of the proximal end side shaft 32 is connected to the proximal end of the hollow shaft 12 and the proximal end is connected to a base portion 42 described later.

The base portion 42 is a portion where a technician operates the dilator 2. The base portion 42 has, for example, a through-hole 42h that communicates with a through-hole 32h of the proximal end side shaft 32 and penetrates from the distal end to the proximal end. The base portion 42 can be configured such that the distal end is connected to the proximal end of the proximal end side shaft 32.

The dilator 2 is configured as described above, so as to be able to prevent the spirally-arranged protruding portion 21 from being detached from the hollow shaft 12 while suppressing a decrease in the flexibility of the tapered portion 121.

### [Third Embodiment]

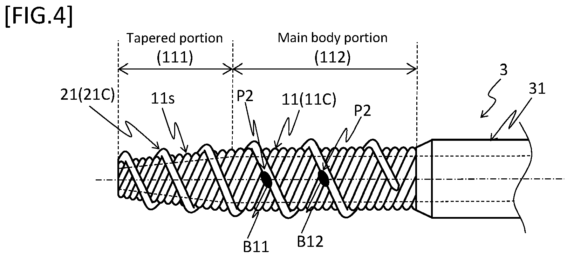

FIG. 4 is a schematic side view illustrating the third embodiment. A dilator 3 is roughly configured of a hollow shaft 11, a spirally-arranged protruding portion 21, a proximal end side shaft 31, and a base portion 41 (not shown), as shown in FIG. 4. The dilator 3 differs from that of the first embodiment in the configuration of the joint part B for joining the hollow shaft 11 and the spirally-arranged protruding portion 21. Since the configurations other than that of the joint part B described below are the same as those of the first embodiment, the same portions/positions are represented by the same reference signs, and detailed descriptions thereof are omitted. Further, the use mode of the dilator 3 is the same as that of the first embodiment, and thus the detailed description thereof is omitted.

The joint part B of the dilator 3 is located at two or more positions, and all of the joint parts B are located only within the range of one round of an arbitrary portion of the spirally-arranged protruding portion 21. The embodiment illustrates the dilator 3, wherein the one round in which the two joint parts B at two positions are provided is located in a midway portion of the coil body 21C (positions P2), and the joint parts B1 1 and B12 are arranged at two locations, the start point and the end point, in the one round.

Since the dilator 3 is configured as described above, wherein the joint part B is located at two or more locations, the spirally-arranged protruding portion 21 can be surely prevented from being detached from the hollow shaft 11, and since all of the joint parts B are located only within the range of one round of the spirally-arranged protruding portion 21, the range of the hollow shaft 11 whose movement is restricted by the spirally-arranged protruding portion 21 is limited, and as a result, a decrease in the flexibility of the tapered portion 111 can be further suppressed.

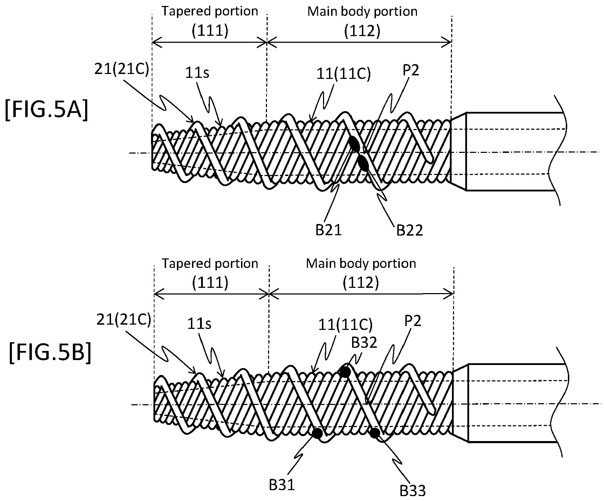

When the joint part B is provided at two or more locations within the range of one round of the spirally-arranged protruding portion 21, the two or more joint parts B may be arranged so as to be adjacent to each other, for example, (see joint parts B21 and B22 in FIG. 5A), or may be arranged at predetermined intervals (equal intervals, etc.) (see joint parts B31, B32, and B33 in FIG. 5B).

### [Fourth Embodiment]

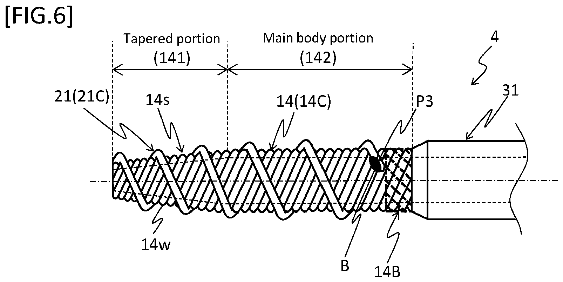

FIG. 6 is a schematic side view illustrating the fourth embodiment. A dilator 4 is roughly configured of a hollow shaft 14, a spirally-arranged protruding portion 21, a proximal end side shaft 31, and a base portion 41 (not shown), as shown in FIG. 6. The dilator 4 differs from that of the first embodiment in the configuration of the hollow shaft 14. Since the configurations of the spirally-arranged protruding portion 21, the proximal end side shaft 31 and the base portion 41 are the same as those of the first embodiment, the same portions/positions are represented by the same reference signs, and detailed descriptions thereof are omitted. Further, since the configurations other than the configuration of the hollow shaft 14 described below and the use mode of the dilator 4 are the same as those of the first embodiment, detailed descriptions thereof are omitted.

The hollow shaft 14 has a tapered portion 141 and a main body portion 142. The tapered portion 141 is a portion in which the outer diameter of the distal end is smaller than the outer diameter of the proximal end. The main body portion 142 is a portion, which has a distal end located at the proximal end of the tapered portion 141, extends toward the proximal end side, and has a constant outer diameter.

In the hollow shaft 14 of this embodiment, the tapered portion 141 and the main body portion 142 are integrally formed. The hollow shaft 14 is configured of a coil body 14C formed of wires 14w that are configured of a plurality of solid wires and are continuously and spirally wound around the long axis.

The wires 14w of the coil body 14C forming the hollow shaft 14 may be twisted wires, may be a bundle (group) of wires, in which a plurality of solid wires are wound in multiple threads in parallel with each other, or may be a combination of twisted wires and a multi-thread group of wires.

Here, the midway portion and/or the proximal end portion of the hollow shaft 14 (coil body 14C) in the longitudinal direction has a binding part in which a plurality of solid wires are integrally bound. The hollow shaft 14 of the embodiment has a binding part 14B in which a plurality of solid wires forming the coil body 14C are integrally bound to each other at the proximal end portion thereof (see FIG. 6).

Examples of a method for forming the binding part 14B include a method for brazing solid wires to each other via a brazing material, a method for directly welding solid wires to each other, and the like. Accordingly, for example, it is possible to prevent the solid wires forming the coil body 14C from being disentangled or the solid wires from being displaced from each other.

The spirally-arranged protruding portion 21 is not joined to the tapered portion 141, but is joined to the main body portion 142 at the joint part B. The joint part B is arranged so as to be located at a position other than positions in the binding part 14B, for example. Specifically, for example, the joint part B may be provided on the outer peripheral surface 14s of the hollow shaft 14 adjacent to the distal end side of the binding part 14B (see FIG. 6), or on the outer peripheral surface 14s of the hollow shaft 14 that is separated from the binding part 14B (not shown).

Since the dilator 4 is configured as described above, the torquability of the hollow shaft 14 can be enhanced by the plurality of solid wires forming the coil body 14C, and since the joint part B is provided on the outer peripheral surface 14s of the hollow shaft 14 other than the binding part 14B having relatively high rigidity created by solid wires binding with each other, it is possible to suppress a steep increase in rigidity (decrease in flexibility) in the longitudinal direction of the binding part 14B.

By the way, as described above, the dilators 1 to 4 of the disclosure can suppress an increase in the rigidity (decrease in flexibility) of the tapered portions 111, 121, and 141. Therefore, the configuration of the disclosure can be suitably applied to a dilator in which the rigidity of the tapered portion itself is higher than the rigidity of the main body portion itself, whereby a further increase in the rigidity of the tapered portion, in which the spirally-arranged protruding portion is provided on the outer peripheral surface, can be suppressed.

Examples of a dilator, in which the rigidity of the tapered portion itself is higher than the rigidity of the main body portion itself, include dilators, in which a hollow shaft is configured of a coil body, such as a dilator wherein the wire of the coil body in the hollow shaft is configured of twisted wires or multi-thread solid wires, a dilator wherein the wire diameter of the tapered portion is the same as or larger than the wire diameter of the main body portion, and a dilator wherein the rigidity of a material forming the tapered portion is larger than the rigidity of a material forming the main body portion.

Note that the disclosed embodiments are not limited to the configurations of the above-described embodiments, but is defined by the terms of the claims and is intended to include any modifications within the scope and meaning equivalent to the terms of the claims.

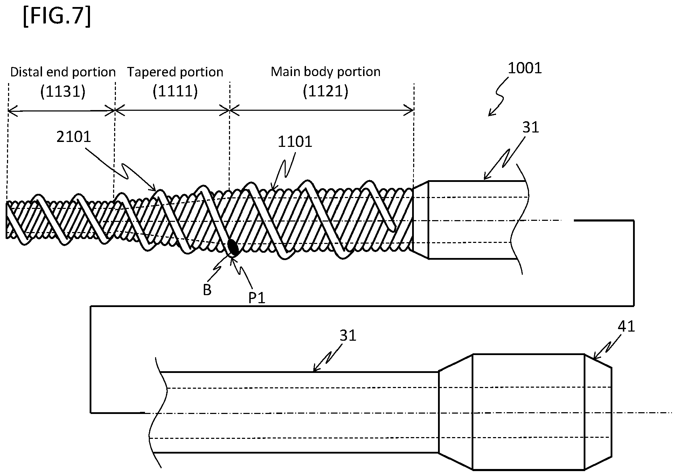

For example, in the above-described embodiments, the dilators 1 to 4 in which the hollow shafts 11, 12, and 14 are configured of the tapered portions 111, 121, and 141 and the main body portions 112, 122, and 142, respectively, are described. However, as shown in FIG. 7, the dilator 1001 may have a distal end portion 1131 which is provided to extend from the distal end of the tapered portion 1111 toward the distal end side and has a constant outer diameter. When a spirally-arranged protruding portion 2101 is provided on the outer peripheral surface of the distal end portion 1131, the distal end portion 1131 and the spirally-arranged protruding portion 2101 are preferably not joined from the viewpoint of suppressing a decrease in the flexibility of the hollow shaft 1101.

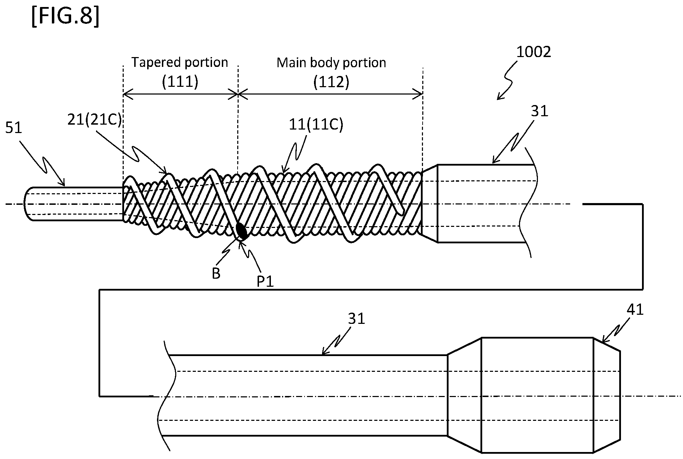

Further, in the above-described embodiments, the dilators 1 to 4 in which the hollow shafts 11, 12, and 14 are configured of the tapered portions 111, 121, and 141 and the main body portions 112, 122, and 142, respectively, are described. However, as shown in FIG. 8, the dilator 1002 may have a hollow-shaped distal tip 51, which is provided to extend from the distal end of the hollow shaft 11 toward the distal end side. The distal tip 51 may be formed of, for example, a soft resin material in order to prevent damage to the tissue.

### [Reference Signs List]

1, 2, 3, 4 Dilator
11, 12, 14 Hollow shaft
111, 121, 141 Tapered portion
112, 122, 142 Main body portion
11C, 14C Coil body
21 Spirally-arranged protruding portion
21C Coil body
21g Gap
21w Wire
B Joint part
   14B Binding part

## Claims

1. A dilator, comprising a hollow shaft having a tapered portion in which the outer diameter of the distal end is smaller than the outer diameter of the proximal end and a main body portion which has a distal end located at the proximal end of the tapered portion, extends toward the proximal end side, and has a constant outer diameter,
wherein: a spirally-arranged protruding portion which is configured of a coil body formed by winding a wire is provided on the outer peripheral surface of the tapered portion and the main body portion;
the spirally-arranged protruding portion has gaps between portions adjacent to each other along the longitudinal direction of the hollow shaft;
and the spirally-arranged protruding portion is not joined to the tapered portion, but is joined to the main body portion at the joint part.

2. The dilator according to claim 1, wherein the joint part is located at a position other than positions in the distal end portion of the main body portion.

3. The dilator according to claim 1 or 2, wherein the joint part is located at only one position.

4. The dilator according to claim 3, wherein the joint part is located at the proximal end portion of the spirally-arranged protruding portion.

5. The dilator according to claim 1 or 2, wherein the joint part is located at two or more positions, and all of the j oint parts are located only within the range of one round of the spirally-arranged protruding portion.

6. The dilator according to any one of claims 1 to 5, wherein the hollow shaft is configured of a coil body formed by winding a wire.

7. The dilator according to claim 6, wherein the wire of the coil body in the hollow shaft is configured of a plurality of solid wires,
a midway portion and/or the proximal end portion of the hollow shaft in the longitudinal direction has a binding part in which the plurality of solid wires are integrally bound,
and the joint part is located at a position other than positions in the binding part.
